# EUROPEAN PATENT SPECIFICATION

(11) **EP 3 897 695 B1**
(45) Date of publication and mention of the grant of the patent: **25.06.2025**
(21) Application number: 19831696.0
(22) Date of filing: 19.12.2019
(51) Int. Cl.: A61K 38/46, C12N 15/861, A61P 3/00

(54) **RECOMBINANT VECTORS FOR THE LONG TERM TREATMENT OF MUCCHOPOLYSACHARIDOSIS**
REKOMBINANTE VEKTOREN ZUR LANGZEITBEHANDLUNG VON MUCCHOPOLYSACHARIDOSE
VECTEURS RECOMBINANT POUR LE TRAITEMENT À LONG TERME DE LA MUCOPOLYSACCHARIDOSE

(30) Priority: 20.12.2018 EP 18382957
(43) Date of publication of application: 27.10.2021
(73) Proprietor: ESTEVE PHARMACEUTICALS, S.A., 08038 Barcelona (ES); Universitat Autònoma de Barcelona, 08193 Cerdanyola del Vallès (ES)
(72) Inventor: BOSCH-TUBERT, Maria-Fatima, 08290 CERDANYOLA DEL VALLES Barcelona (ES); MARCO-COSTA, Sara, 17190 SALT Girona (ES); MORTE-PEREZ, Adelaida, 08193 CERDANYOLA DEL VALLES Barcelona (ES); PLATA-SALAMAN, Carlos-Ramon, 08021 BARCELONA Barcelona (ES)
(74) Representative: Pons
(86) International application number: PCT/EP2019/086227
(87) International publication number: WO 2020/127678

(56) References cited:
- EP-A1- 2 394 667
- EP-A1- 2 492 347
- WO-A1-2015/173308
- WO-A1-2018/209317
- ANONYMOUS: "ESTEVE announces the start of a natural history study in patients with Sanfilippo Syndrome Type A (MPSIIIA)", 31 July 2014 (2014-07-31), XP093234500, Retrieved from the Internet <URL:https://www.esteve.com/GetFichero.do?idi=3&zon=3&con=3494&fichero=Ar_3_3_3494_DR_1.pdf>
- VIRGINIA HAURIGOT ET AL: "Whole body correction of mucopolysaccharidosis IIIA by intracerebrospinal fluid gene therapy", JOURNAL OF CLINICAL INVESTIGATION, vol. 123, no. 8, 1 July 2013 (2013-07-01), pages 3254 - 3271, XP055210967, ISSN: 0021-9738, DOI: 10.1172/JCI66778
- MARC TARDIEU ET AL: "Intracerebral Administration of Adeno-Associated Viral Vector Serotype rh.10 Carrying Human SGSH and SUMF1 cDNAs in Children with Mucopolysaccharidosis Type IIIA Disease: Results of a Phase I/II Trial", HUMAN GENE THERAPY, vol. 25, no. 6, 1 June 2014 (2014-06-01), US, pages 506 - 516, XP055467864, ISSN: 1043-0342, DOI: 10.1089/hum.2013.238
- ALBERT RIBERA ET AL: "Biochemical, histological and functional correction of mucopolysaccharidosis Type IIIB by intra-cerebrospinal fluid gene therapy", HUMAN MOLECULAR GENETICS, vol. 24, no. 7, 18 December 2014 (2014-12-18), gb, pages 2078 - 2095, XP055571308, ISSN: 0964-6906, DOI: 10.1093/hmg/ddu727

## Description

### FIELD OF THE INVENTION

The present invention relates to vectors and nucleic acid sequences for the treatment of mucopolysaccharidoses (MPS), and in particular, for the treatment of mucopolysaccharidoses type III A or Sanfilippo A syndrome.

### BACKGROUND OF THE INVENTION

Lysosomal storage diseases (LSDs) are caused by genetic defects that affect one or more lysosomal enzymes. These genetic diseases result generally from a deficiency in a particular enzyme activity present in the lysosome. To a lesser extent, these diseases may be due to deficiencies in proteins involved in lysosomal biogenesis.

LSDs are individually rare, although as a group these disorders are relatively common in the general population. The combined prevalence of LSDs is approximately 1 per 5,000 live births. However, some groups within the general population are particularly afflicted by a high occurrence of LSDs. For instance, the prevalence rates of the Gaucher and Tay-Sachs diseases in descendants from Jewish Central and Eastern European (Ashkenazi) individuals is 1 per 600 and 1 per 3,900 births, respectively. The Finnish population is also afflicted by an uncommonly high LSDs prevalence rate.

Type III mucopolysaccharidoses (MPSIII), known collectively as Sanfilippo syndrome, are LSDs caused by a deficiency in one of the enzymes involved in the degradation of heparan sulfate, leading to its pathological accumulation. MPSIII is classified into four subtypes depending on the enzyme deficiency. Loss of sulfamidase activity causes subtype IIIA and has been reported to be the most severe, with the earliest disease onset and shortest survival. Symptoms of MPSIIIA occur in the first years of life, and are characterized by severe neurodegeneration that leads to deep mental retardation, aggressiveness, hyperactivity, and sleep alterations. Patients progressively lose the capacity of speech, swallow, and basic motor coordination. In addition to the neurological symptoms, MPSIIIA patients suffer non-neurological alterations, including hepato- and splenomegaly, skeletal and joint malformations, as well as frequent diarrhoea and respiratory tract infections. The progressive worsening of symptoms results in the death of the patient during adolescence.

There is no cure for MPSIIIA currently and, therefore, existing treatments are aimed at controlling symptoms of the disease in order to improve the poor quality of life of the patients. MPS disorders can be treated by bone marrow transplantation or enzyme replacement therapy (ERT). Both approaches rely in the endocytosis of lysosomal enzymes from extracellular medium and their targeting to lysosomes via the mannose-6-phosphate receptor (M6PR) present at the cell membrane. Nevertheless, bone marrow transplantation has demonstrated to be inefficient in the treatment of MPSIII patients. ERT has been extensively proven to be effective in counteracting the non-neurological accumulation in other lysosomal storage diseases, including MPSI, II and VI. In addition to the high cost of these treatments, it has been shown that ERT does not result in correction or preservation of neuronal function due to the insufficient delivery of the exogenously provided enzyme through the blood-brain barrier (BBB). Also, it has been demonstrated that high-dose ERT is partially successful in clearing CNS storage in MPS VII, possibly due to the saturation of M6PR and mannose receptors that lead to a longer half-life of the protein in circulation (Vogler C, et al., Proc. Natl. Acad. Sci. USA 2005; 102:14777-14782).

Intra-cerebral and intra-cerebrospinal fluid (CSF) delivery of the enzyme have also been proved to be efficient in reducing CNS pathology in MPS IIIA mice (Hemsley K, et al., Genes Brain Behav. 2008; 53(2):161-8 and Savas P, et al., Mol. Genet. Metab. 2004; 82:273-285). However, this approach is highly invasive due to the need for multiple repeated injections and could increase the risk of damage and/or infections in the brain.

Given the limitations of current therapeutic options for MPSIII, alternative approaches are needed. Gene transfer could provide the means to achieve a permanent production of the missing enzyme from a single intervention. Adeno-associated vectors (AAV) are rapidly emerging as the vector of choice for many gene therapy applications, due to their high transduction efficiency and their lack of pathogenicity. AAV vectors efficiently transduce post-mitotic cells and several preclinical and clinical studies demonstrated the potential of AAV vector-mediated gene transfer to efficiently drive sustained expression of therapeutic transgenes for a variety of diseases.

Fraldi et al (Biochen. J, 2007; 403, 305-312) demonstrated the efficacy of gene transfer in only newborn MPSIIIA mice. No experiments were reported in older mice. Since MPSIIIA is usually diagnosed after 3-4 years of age, the newborn animal model is not adequate for predicting the effects of this treatment in human beings.

Haurigot et al (J Clin Invest. 2013 Aug 1; 123(8): 3254-3271) demonstrated that upon delivery of AAV9 vectors encoding for human sulfamidase to the CSF of dogs, activity of the enzyme in the CSF peaked within 2-3 weeks. This increment was, however, accompanied by signs of CNS inflammation, such as a rise in protein content and white blood cells counts in the CSF of injected animals. Subsequently, detection of the activity of the transgene in the CSF dropped, and so did markers of inflammation.

In EP2394667A1 it is disclosed AAV expression cassettes generated by cloning the cDNA of an optimized human sulfamidase (SEQ ID NO: 1) under the control of the ubiquitous CAG promoter (hybrid of chicken β-actin promoter and CMV enhancer) into single-stranded AAV backbone plasmids. The resultant plasmid contains cDNA of optimized human sulfamidase (SEQ ID NO: 1) under the control of the ubiquitous CAG promoter and flanked by AAV2 ITRs.

On the other hand, high transgene overexpression in certain cell types could potentially lead to cell dysfunction and inflammation, and transgene-associated toxicities have been reported following delivery of genes to the CNS (Golebiowski et al, Hum Gene Ther, 2017; Jun 1, 28(6): 510-522).

There is thus a need for new approaches for the long term treatment of neurological diseases in humans, in particular lysosomal storage diseases (LSD), which are safe and show no adverse events.

### SUMMARY OF THE INVENTION

The inventors have found that, a single administration of the pharmaceutical composition of the invention into the cerebrospinal fluid (CSF) of MPSIIIA patients surprisingly, was able to produce steady high levels of SGSH activity in the CSF and other tissues of the patients in the absence of any adverse events. Altogether, the results confirm that is possible to achieve long-term transgene expression in the CNS in humans using this gene transfer approach.

Furthermore, the inventors have demonstrated the utility of measuring sulfamidase enzymatic activity in a sample of the subject, in particular in the CSF, as a tool to monitor gene transfer efficacy.

Thus, in a first aspect, the invention refers to a pharmaceutical composition comprising a recombinant adeno-associated viral vector comprising a nucleotide sequence as set for in SEQ ID NO: 1 encoding sulfamidase protein of SEQ ID NO: 2 for use as long term treatment, wherein the long term treatment shows an effect that persists for at least three months, of Sanfilippo syndrome type A in a human subject, by means of one single intracerebroventricular administration of the pharmaceutical composition and resulting in prolonged and sustained production of the sulfamidase protein.

In a second aspect, the invention refers to a method for evaluating and/or monitoring the efficacy of a long term treatment, wherein the long term treatment shows an effect that persists for at least three months, of Sanfilippo syndrome type A in a human subject after one single intracerebroventricular administration of a pharmaceutical composition comprising a recombinant adeno-associated viral vector comprising a nucleotide sequence as set for in SEQ ID NO: 1 encoding sulfamidase protein of SEQ ID NO: 2, comprising measuring sulfamidase enzymatic activity in a sample of the treated subject at least three months after administration.

### DEFINITIONS

A "vector" as used herein refers to a macromolecule or association of macromolecules that comprises or associates with a polynucleotide and which can be used to mediate delivery of the polynucleotide to a cell. Illustrative vectors include, for example, plasmids, viral vectors, liposomes and other gene delivery vehicles.

The terms "adeno-associated virus", "AAV virus", "AAV virion," "AAV viral particle" and "AAV particle", used as synonyms herein, refer to a viral particle composed of at least one capsid protein of AAV (preferably composed of all capsid proteins of a particular AAV serotype) and an encapsulated polynucleotide corresponding to the AAV genome. The wild-type AAV refers to a virus that belongs to the genus Dependovirus, family Parvoviridae. The wild-type AAV genome is approximately 4.7 Kb in length and consists of a single stranded deoxyribonucleic acid (ssDNA) that can be positive or negative-sensed. The wild-type genome includes inverted terminal repeats (ITR) at both ends of the DNA strand, and three open reading frames (ORFs). The ORF rep encodes for four Rep proteins necessary for AAV lifecycle. The ORF cap contains nucleotide sequences encoding capsid proteins: VP1, VP2 and VP3, which interact to form a capsid of icosahedral symmetry. Finally, the AAP ORF, which overlaps with the Cap ORF, encodes for the AAP protein that appears to promote capsid assembly. If the particle comprises a heterologous polynucleotide (i.e. a polynucleotide different from a wild-type AAV genome, such as a transgene to be delivered to a mammalian cell) flanked by AAV ITRs, then it is typically known as "AAV vector particle" or "AAV viral vector" or "AAV vector". The invention also encompasses the use of double stranded AAV also called dsAAV or scAAV.

The term "adeno-associated virus ITRs" or "AAV ITRs", as used herein, refers to the inverted terminal repeats present at both ends of the DNA strand of the genome of an AAV. The ITR sequences are required for efficient multiplication of the AAV genome. Another property of these sequences is their ability to form a hairpin. This characteristic contributes to their self-priming, which allows the primase-independent synthesis of the second DNA strand. The ITRs have also been shown to be required for both integration of the wild-type AAV DNA into the host cell genome (e.g. in the human 19^{th} chromosome for serotype 2 AAV) and rescue from it, as well as for efficient encapsidation of the AAV DNA into a fully assembled, deoxyribonuclease-resistant AAV particle. The ITR sequences are about 145 bp in length. Preferably, the entire sequences of the ITRs are used in the genome of the AAV viral vector, although some degree of minor modification of these sequences is permissible. A wild-type ITR sequence may be altered by insertion, deletion or truncation, as long as the ITR mediates the desired functions, e.g. replication, nicking, virus packaging, integration, and/or provirus rescue. Procedures for modifying these ITR sequences are well known in the art. The ITR may be from any wild-type AAV, including but not limited to serotypes 1, 2, 3, 4, 5, 6, 7, 8, 9, 10, 11 or 12 or any other AAV known or later discovered. The AAV comprises two ITRs, which may be the same or different. Further, the two AAV ITRs can be from the same AAV serotype as the AAV capsid, or can be different. In a preferred embodiment, the 5' and 3' AAV ITRs derive from AAVI, AAV2, AAV4, AAV5, AAV7, AAV8 and/or AAV9 Preferably ITRs are from AAV2, AAV8 and/or AAV9 being AAV2 the most preferred. In one embodiment, the AAV2 ITRs are selected to generate a pseudotyped AAV (i.e. an AAV having capsid and ITRs derived from different serotypes).

The expression "recombinant viral genome", as used herein, refers to an AAV genome in which at least one extraneous polynucleotide is inserted into the naturally occurring AAV genome. The genome of the AAV according to the invention typically comprises the cis-acting 5' and 3' inverted terminal repeat sequences (ITRs) and an expression cassette. An "rAAV vector" as used herein refers to an AAV vector comprising a polynucleotide sequence not of AAV origin (i.e., a polynucleotide heterologous to AAV), typically a sequence of interest for the genetic transformation of a cell. In preferred vector constructs of this invention, the heterologous polynucleotide is flanked by two AAV inverted terminal repeat sequences (ITRs).

An "AAV virus" or "AAV viral particle" refers to a viral particle composed of at least one AAV capsid protein (preferably by all of the capsid proteins of a wild-type AAV) and an encapsidated polynucleotide. If the particle comprises a heterologous polynucleotide (i.e. a polynucleotide other than a wild-type AAV genome such as a transgene to be delivered to a mammalian cell), it is typically referred to as "rAAV vector particle", "rAAV product" or "rAAV vector".

"Packaging" refers to a series of intracellular events that result in the assembly and encapsidation of an AAV particle.

AAV "rep" and "cap" genes refer to polynucleotide sequences encoding replication and encapsidation proteins of adeno-associated virus. They have been found in all AAV serotypes examined, and are described below and in the art. AAV rep and cap are referred to herein as AAV "packaging genes".

The term "CAG promoter" refers to the combination formed by the cytomegalovirus early enhancer element and chicken β-actin promoter (See Alexopoulou A, et al., BMC Cell Biology 2008; 9(2): 1-11).

The term "polynucleotide" refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a polynucleotide encompasses both the double-stranded form and each of two complementary single-stranded forms known or predicted to make up the double-stranded form.

A "gene" refers to a polynucleotide containing at least one open reading frame that is capable of encoding a particular protein after being transcribed and translated.

The term "nucleotide sequence" refers to a nucleic acid molecule, either DNA or RNA, containing deoxyribonucleotides or ribonucleotides. The nucleic acid may be double stranded, single stranded, or contain portions of both double stranded or single stranded sequence.

The term "codify" refers to the genetic code that determines how a nucleotide sequence is translated into a polypeptide or a protein. The order of the nucleotides in a sequence determines the order of amino acids along a polypeptide or a protein.

"Recombinant", as applied to a polynucleotide means that the polynucleotide is the product of various combinations of cloning, restriction or ligation steps, and other procedures that result in a construct that is distinct from a polynucleotide found in nature. A recombinant virus is a viral particle comprising a recombinant polynucleotide. The terms respectively include replicates of the original polynucleotide construct and progeny of the original virus construct.

A "control element" or "control sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Control elements known in the art include, for example, transcriptional regulatory sequences such as promoters and enhancers. A promoter is a DNA region capable under certain conditions of binding RNA polymerase and initiating transcription of a coding region usually located downstream (in the 3' direction) from the promoter.

"Operatively linked" or "operably linked" refers to a juxtaposition of genetic elements, wherein the elements are in a relationship permitting them to operate in the expected manner. For instance, a promoter is operatively linked to a coding region if the promoter helps initiate transcription of the coding sequence. There may be intervening residues between the promoter and coding region so long as this functional relationship is maintained.

An "expression vector" is a vector comprising a region which encodes a polypeptide of interest, and is used for effecting the expression of the protein in an intended target cell. An expression vector also comprises control elements operatively linked to the encoding region to facilitate expression of the protein in the target. The combination of control elements and a gene or genes to which they are operably linked for expression is sometimes referred to as an "expression cassette," a large number of which are known and available in the art or can be readily constructed from components that are available in the art.

"Heterologous" means derived from a genotypically distinct entity from that of the rest of the entity to which it is being compared. For example, a polynucleotide introduced by genetic engineering techniques into a plasmid or vector derived from a different species is a heterologous polynucleotide. A promoter removed from its native coding sequence and operatively linked to a coding sequence with which it is not naturally found linked is a heterologous promoter.

"Genetic alteration" refers to a process wherein a genetic element is introduced into a cell other than by mitosis or meiosis. The element may be heterologous to the cell, or it may be an additional copy or improved version of an element already present in the cell. Genetic alteration may be effected, for example, by transfecting a cell with a recombinant plasmid or other polynucleotide through any process known in the art, such as electroporation, calcium phosphate precipitation, or contacting with a polynucleotide-liposome complex. Genetic alteration may also be effected, for example, by transduction or infection with a DNA or RNA virus or viral vector. Preferably, the genetic element is introduced into a chromosome or mini-chromosome in the cell; but any alteration that changes the phenotype and/or genotype of the cell and its progeny is included in this term.

The terms "polypeptide", "peptide" and "protein" are used interchangeably herein to refer to polymers of amino acids of any length. The terms also encompass an amino acid polymer that has been modified; for example, disulfide bond formation, glycosylation, lipidation, or conjugation with a labeling component.

An "isolated" plasmid, virus, or other substance refers to a preparation of the substance devoid of at least some of the other components that may also be present where the substance or a similar substance naturally occurs or is initially prepared from. Thus, for example, an isolated substance may be prepared by using a purification technique to enrich it from a source mixture. Enrichment can be measured on an absolute basis, such as weight per volume of solution, or it can be measured in relation to a second, potentially interfering substance present in the source mixture. Increasing enrichments of the embodiments of this invention are increasingly more preferred. Thus, for example, a 2-fold enrichment is preferred, 10-fold enrichment is more preferred, 100-fold enrichment is more preferred, 1000-fold enrichment is even more preferred.

An "individual" or "subject" treated in accordance with this invention refers to vertebrates, particularly members of a mammalian species, and includes but is not limited to domestic animals, sports animals, and primates, including humans.

"Treatment" of an individual or a cell is any type of intervention in an attempt to alter the natural course of the individual or cell at the time the treatment is initiated. For example, treatment of an individual may be undertaken to decrease or limit the pathology caused by any pathological condition, including (but not limited to) an inherited or induced genetic deficiency, infection by a viral, bacterial, or parasitic organism, a neoplastic or aplastic condition, or an immune system dysfunction such as autoimmunity or immunosuppression. Treatment includes (but is not limited to) administration of a composition, such as a pharmaceutical composition, and administration of compatible cells that have been treated with a composition. Treatment may be performed either prophylactically or therapeutically; that is, either prior or subsequent to the initiation of a pathologic event or contact with an etiologic agent.

The term "effective amount" refers to an amount of a substance sufficient to achieve the intended purpose. For example, an effective amount of an expression vector to increase sulfamidase activity is an amount sufficient to reduce glycosaminoglycan accumulation. A "therapeutically effective amount" of an expression vector to treat a disease or disorder is an amount of the expression vector sufficient to reduce or remove the symptoms of the disease or disorder. The effective amount of a given substance will vary with factors such as the nature of the substance, the route of administration, the size and species of the animal to receive the substance and the purpose of giving the substance. The effective amount in each individual case may be determined empirically by a skilled artisan according to established methods in the art.

The term "gene therapy" refers to the transfer of genetic material (e.g. DNA or RNA) of interest into a host to treat or prevent a genetic or acquired disease or condition. The genetic material of interest encodes a product (e.g. a protein polypeptide, peptide or functional RNA) whose production *in vivo* is desired. For example, the genetic material of interest can encode an enzyme, hormone, receptor, or polypeptide of therapeutic value.

### DETAILED DESCRIPTION OF THE INVENTION

Delivery of adeno-associated viral (AAV) vectors into the cerebrospinal fluid (CSF) can achieve gene transfer to cells throughout the brain and spinal cord, potentially making many neurological diseases, in particular, lysosomal storage diseases (LSD), tractable gene therapy targets.

While studies in small animal models have provided proof of concept, and experiments in large animals have demonstrated feasibility in bigger brains, there is no information on the long-term safety or the durability of the effect in humans. The limited lifespan of these animal species precludes long-term evaluations of efficacy and safety.

The inventors have found that surprisingly, a single dose of rAAV9 vectors encoding human sulfamidase (SGSH) administered intra-CSF to Sanfilippo syndrome type A patients results in long time detection of sulfamidase activity in the CSF and other tissues of the subjects. The inventors herein shows that the method of the invention is safe and results in steady high levels of SGSH activity in the tissues, including serum, in the absence of any adverse events.

Thus, in a first aspect the invention refers to a pharmaceutical composition comprising a recombinant adeno-associated viral vector comprising a nucleotide sequence as set for in SEQ ID NO: 1 encoding sulfamidase protein of SEQ ID NO: 2 for use as long term treatment, wherein the long term treatment shows an effect that persists for at least three months, of Sanfilippo syndrome type A in a human subject, by means of one single intracerebroventricular administration of the pharmaceutical composition and resulting in prolonged and sustained production of the sulfamidase protein.

Identifying the optimal route of CSF access for AAV delivery is a critical step in clinical practice. Intrathecal delivery methods enable the administration of soluble therapeutics directly into the central nervous system (CNS). Intrathecal delivery methods include intracerebroventricular (ICV), intrathecal-lumbar and intracisternal routes. The ICV route enables the administration of drugs into a lateral cerebral ventricle via an implanted device (reservoir and catheter). This delivery route, which may also be referred to as intraventricular administration, has been used worldwide for decades to treat pediatric and adult patients with a broad range of CNS disorders. The ICV route of administration instills therapy into the cerebral ventricles. In addition to the ICV route, intrathecal delivery methods include single or repeated intrathecal lumbar (IT-L) injections, in which agents are directly administered into the cerebrospinal fluid (CSF) by puncturing the membranes surrounding the spinal cord. Intrathecal routes of administration allow therapies to bypass the blood-brain barrier (BBB) and are commonly used to treat a variety of diseases in pediatric and adult patients. ICV devices allow administration of drugs either directly into the CSF or by interstitial infusion (with convection). Whereas interstitial infusion by convection reaches a perimeter from the implanted point source within the parenchyma, injection/infusion into the CSF will distribute throughout the whole ventricular system and the external CSF spaces like the basal cisterns and over the convexity.

In another particular embodiment, said composition is for use as long term treatment of patients with or susceptible to Sanfilippo syndrome type A.

The term "long term" as used herein refers to a treatment which shows great duration in time. According to the present invention, after a single administration of the pharmaceutical composition of the invention, results in an effect which is prolonged in time, said effect persists for at least least 3 months, preferably at least 6 months, at least 9 months, at least 12 months, at least 18 months, at least 24 months, at least 30 months, at least 36 months, at least 42 months, at least 48 months, at least 60 months.

As mentioned above, symptoms of MPSIIIA occur in the first years of life, being pre-natal and early stages of post-natal development usually normal.

The order of events from heparan sulfate accumulation through downstream changes in the levels of other biomolecules within the cell and ultimately the clinical symptoms of the disease, particularly with respect to CNS degeneration, is probably the least understood aspect of the syndrome.

It is also disclosed herein the treatment of a subject who is susceptible to Sanfilippo syndrome type A, i.e. a subject with confirmed MPSIIIA, for example, by genotype analysis, but showing no clinical symptoms of the disease yet. Thus, in the present disclosure, said human subject may show no clinical symptoms of Sanfilippo A syndrome. Thus, said subject is in the early phases of the disease.

More particularly, said subject shows no clinical symptoms of the disease but shows a mutation at least in one of the alleles of the sulfamidase gene sequence. In this disclosure, said mutation is a missense mutation, a deletion, an insertion, a truncation or a nonsense mutation.

Alternatively or additionally, the subject shows high levels of GAGs, in particular, heparan sulfate, measured in a fluid sample of said subject with no clinical symptoms of the disease. Also, in this disclosure, the subject may show deficiency in sulfamidase enzyme activity. In this disclosure, the subject may show no detectable levels of sulfamidase activity in a sample of said subject.

The detection of GAGs in a sample of a suspected MPS IIIA patient, such as a urine sample, is often the first biochemical step in diagnosis.

There are several methods well known for the skilled person in the art for GAG quantification in a sample from a subject. As an example, urinary GAG quantification involves the use of dimethylmethylene blue. Here, dimethylmethylene blue associates with sulfated GAG and the absorbance of the complex at a wavelength of 520 nm can be measured on a spectrophotometer. An elevation in urinary GAG will suggest a form of MPS. Subsequent analysis can permit a further characterization of the accumulating product. High-resolution cellulose acetate electrophoresis of urinary samples can separate and identify distinct GAGs, thus providing an indication of MPS III from other forms. Additionally, qualitative gradient polyacrylamide gel electrophoretic separation can confirm increased secretion of particular heparan sulfate chains, thus diagnosing the particular MPS III subtype.

Further methods well known in the art comprising tandem mass spectrometry can also be used. Additionally, extraction and depolymerization of heparan sulfate using methanolysis under acidic conditions, and analysis of the subsequent desulfated disaccharide reaction products by liquid chromatography/tandem mass spectrometry can be used. A liquid chromatography/tandem mass spectrometrybased technique that exploits the unique nature of the non-reducing end residue of the accumulated GAG upon the deficiency of a particular enzyme in the pathway has also been described in the literature.

Thus, in a particular embodiment, GAG accumulation, in particular, heparan sulfate accumulation above standard levels, has been detected in a sample from said subject.

DNA samples from the subject are normally sequenced and crossreferenced to the standard wild-type sequence and known disease-causing mutations and polymorphisms. This is achieved by employing oligonucleotide primers that span the intron/exon junctions of the exons of the gene of interest using the polymerase chain reaction with genomic DNA as the template.

DNA samples can be obtained from the chorionic villus at 9-10 weeks gestation, i.e. during prenatal period. Also, cultured cells from amniocentesis can be tested for MPSIIIA causing mutations via genomic DNA sequencing or enzymatic activity assays. Increased heparan sulfate, can also been detected using electrophoresis in amniotic fluid taken from a pregnancy involving an MPSIIIA fetus.

According to the present invention, the pharmaceutical composition of the invention contains recombinant AAV vectors. The AAV genome is built of single-stranded deoxyribonucleic acid (ssDNA). AAV infect humans but are nonpathogenic (i.e. do not cause a disease). They can infect dividing and non-dividing cells, and their tropism changes depending on the serotype. The serotype is the classification of the viruses in groups, depending on their capsid antigens. The serotype of the AAV, determined by its capsid proteins, defines the virus tropism and allows its entry into a specific cell type.

AAV according to the present invention include any serotype of the AAV known serotypes. In general, the different serotypes of AAV have genomic sequences with a significant homology, providing an identical series of genetic functions, produce virions that are essentially equivalent in physical and functional terms, and replicate and assemble through practically identical mechanisms. In particular, the AAV of the present invention may belong to the serotype 1 of AAV (AAV1), AAV2, AAV3 (including types 3A and 3B), AAV4, AAV5, AAV6, AAV7, AAV8, AAV9, AAV10, AAV11, avian AAV, bovine AAV, canine AAV, equine AAV, ovine AAV, and any other AAV. Examples of the sequences of the genome of the different AAV serotypes may be found in the literature or in public databases such as GenBank. See GenBank accession numbers AF028704.1 (AAV6), NC006260 (AAV7), NC006261 (AAV8), and AX753250.1 (AAV9). In a preferred embodiment, the AAV vector of the invention is of a serotype selected from the group consisting of the AAV2, AAV5, AAV7, AAV8, AAV9, AAV10 and AAVrh10 serotypes. In a preferred embodiment, said AAV vector is of serotype 9, AAV9 or serotype 8, AAV8. More preferably, the AAV vector is AAV9. The production of the adeno-associated vector particles comprised in the pharmaceutical composition of the invention is described below.

It is also disclosed herein, a pharmaceutical composition comprising a recombinant AAV vector comprising a nucleotide sequence as set for in SEQ ID NO: 1 for use in a method comprising a step of administering the composition intra-CSF to a subject suffering from or susceptible to Sanfilippo syndrome type A wherein said composition is for single administration for long term treatment of Sanfilippo syndrome type A resulting in prolonged and sustained production of the sulfamidase protein in the subject.

Pharmaceutical compositions can be supplied as liquid solutions or suspensions, as emulsions, or as solid forms suitable for dissolution or suspension in liquid prior to use. Although not required, pharmaceutical compositions may optionally be supplied in unit dosage form suitable for administration of a precise amount. In a preferred embodiment, the composition of the invention is formulated as a suspension for injection.

An effective amount of virus is administered, depending on the objectives of treatment. Where a low percentage of transduction can cure a genetic deficiency, then the objective of treatment is generally to meet or exceed this level of transduction. In some instances, this level of transduction can be achieved by transduction of only about 1 to 5% of the target cells, but is more typically 20% of the cells of the desired tissue type, usually at least about 50%, preferably at least about 80%, more preferably at least about 95%, and even more preferably at least about 99% of the cells of the desired tissue type.

The pharmaceutical composition according to the present invention is suitable for single intra-CSF administration, in particular, for intrathecal administration, more particularly, for intracerebroventricular administration.

Schedules and dosages for the administration of the pharmaceutical composition according to the present invention can be determined in accordance to dosage protocols known in the art.

In a particular embodiment, the pharmaceutical composition for use according to the invention is administered at a single dose of 1x10¹² to 1x10¹⁵ vg/Kg. In a more particular embodiment, said composition is administered at a single dose of 1x10¹³ to 1x10¹⁵ vg/Kg. In a preferred embodiment, said composition is administered at a single dose of 1x10¹³ to 3x10¹⁴ vg/Kg. In a more preferred embodiment, said composition is administered at a single dose of 3.4 x 10¹³ vg/Kg to 1.4 x 10¹⁴ vg/Kg.

Storage of GAGs in cells of patients suffering from MPS results in a progressive damage of the affected tissues. Sanfilippo type A is characterized by lysosomal storage of heparan sulfate. According to the invention, after a single administration of the pharmaceutical composition of the invention containing the rAAV product, GAGs levels, in particular heparan sulfate levels, are reduced. Moreover, said reduction is sustained in time.

Thus, in a particular embodiment of the invention, the percentage of reduction of glycosaminoglycans levels, in particular heparan sufate, in a sample of said patient such as for example cerebrospinal fluid (CSF), urine and/or serum is of at least 10%, at least 15%, at least 20%, at least 25%, at least 30%, at least 40%, at least 50%, at least 60%, at least 70%, at least 80%, at least 90%, or at least 99% compared to baseline levels, i.e. levels of GAGs, in particular, heparan sulfate, in a sample of said subject measured before treatment. Moreover, in a preferred embodiment, said reduction achieves standard levels and are prolonged and sustained in time. The term "standard levels" as used herein refers to GAGs levels, in particular, heparan sulfate levels, found in a sample from a normal subject, i.e. a subject not having Sanfilippo A syndrome.

In another particular embodiment, the percentage of reduction of heparan sulfate levels in a sample of said subject is of at least 10%, at least 15%, at least 20%, at least 25%, at least 30%, or at least 40% compared to baseline levels and is maintained for at least 3 months after treatment. In a more particular embodiment, the percentage of reduction of heparan sulfate levels in a sample of said subject is of at least 10%, at least 15%, at least 20%, at least 25%, at least 30%, at least 40%, or at least 50%, compared to baseline levels and is maintained at least 6 months after treatment. In a more particular embodiment, the percentage of reduction of heparan sulfate levels in a sample of said subject is of at least 20%, at least 25%, at 30%, at least 40%, or at least 50%, compared to baseline levels and is maintained at least 12 months after treatment. Moreover, in a preferred embodiment, said reduction achieves standard levels and are prolonged and sustained in time.

The inventors have found that a single administration of the pharmaceutical composition of the invention resulted in a long-term, stable increase in sulfamidase activity in CSF and other tissues. Indeed, the inventors have shown that there is a long-term, stable increase in sulfamidase activity in the subject. Thus, in a particular embodiment, the invention refers a pharmaceutical composition according to the invention for use as long term treatment of Sanfilippo syndrome type A resulting in an increase of enzyme activity levels compared to baseline levels in a sample of said subject, i.e. levels before treatment of said patient. In a particular embodiment, the pharmaceutical composition for use according to the invention results in an increase of enzyme activity levels in a sample of said subject is of at least two fold compared to baseline levels when measured at least 3 months after treatment. Preferably, said sample is an intra-cerebrospinal fluid (CSF) sample, a plasma or serum sample or a leukocyte sample.

In a more particular embodiment, the pharmaceutical composition for use according to the invention results in an increase of enzyme activity of at least 2 fold, at least 2.5 fold, at least 3 fold, at least 3.5 fold, at least 4 fold, at least 4.5 fold, at least 5 fold, at least 5.5 fold, at least 6 fold, at least 6.5, at least 7 fold, or at least 8 fold compared to the patient's baseline levels, i.e. levels measured before treatment, when measured in a sample of said subject.

In another particular embodiment, the percentage of increase of enzyme activity levels in a sample of said subject at 6 months after treatment is of at least 2 fold, at least 2.5 fold, at least 3 fold, at least 3.5 fold, at least 4 fold, at least 4.5 fold, at least 5 fold, at least 5.5 fold, at least 6 fold, at least 6.5, at least 7 fold, or at least 8 fold compared to patient's baseline levels, i.e. levels measured before treatment.

In a more particular embodiment, the percentage of increase of enzyme activity levels in a sample of said subject at 12 months after treatment is of at least 2 fold, at least 2.5 fold, at least 3 fold, at least 3.5 fold, at least 4 fold, at least 4.5 fold, at least 5 fold, at least 5.5 fold, at least 6 fold, at least 6.5, at least 7 fold, or at least 8 fold compared to patient's baseline levels.

In a preferred embodiment, the increase of enzyme activity levels achieves standard levels, i.e. levels comparable to a control subject, and are prolonged and sustained in time. In another preferred embodiment, enzyme activity levels are superior to standard levels, i.e. levels greater to a control subject, and are prolonged and sustained in time.

In a particular embodiment, the pharmaceutical composition of the invention achieves a therapeutic concentration of the enzyme within the affected cells and tissues, in particular the brain.

As mentioned above, the inventors have found that a single administration of the pharmaceutical composition of the invention, wherein said administration is an intra-CSF administration, resulted in a long-term, stable increase in sulfamidase activity in CSF and other tissues, such as serum, in the absence of any safety concerns. Thus, in another particular embodiment, the invention refers to a pharmaceutical composition according to the invention wherein the administration of the composition to the subject does not result in any substantial adverse effect.

According to the present invention, by monitoring of biochemical and hematological parameters in CSF and blood samples, clinical and neurological evaluations and imaging of target organs the good tolerability and long-term safety of this gene transfer approach has been demonstrated.

Thus, in another aspect, the invention refers to a method for evaluating and/or monitoring the efficacy of a long term treatment, wherein the long term treatment shows an effect that persists for at least three months, of Sanfilippo syndrome type A in a human subject after one single intracerebroventricular administration of a pharmaceutical composition comprising a recombinant adeno-associated viral vector comprising a nucleotide sequence as set for in SEQ ID NO: 1 encoding sulfamidase protein of SEQ ID NO: 2, comprising measuring sulfamidase enzymatic activity in a sample of the treated subject at least three months after administration. In a particular embodiment the sample is a CSF sample or a serum sample. Said measuring is performed at least three months after administration, preferably at least six months after administration, at least nine months after administration, at least twelve months after administration, at least fifteen months after administration, at least eighteen months after administration, at least twentyfour months after administration, or at least thirty-six months after administration.

Methods for the production of recombinant AAV (rAAV) vectors are well known in the art.

In a particular embodiment, rAAV are produced transiently in mammalian cells, for example in HEK293 cells, by co-transfecting two or three plasmids containing AAV genes, adenovirus helper genes, and a vector genome. Generation of a rAAV plasmid involves replacing a majority of the AAV's genome with a desired transgene and providing the viral genes that are essential for virus packaging in-trans on a separate plasmid. In a particular embodiment, AAV expression cassettes are generated by cloning the cDNA of optimized human sulfamidase (SEQ ID NO: 1) under the control of the ubiquitous CAG promoter (hybrid of chicken β-actin promoter and CMV enhancer) into single-stranded AAV backbone plasmids. In a preferred embodiment, said plasmid is pAAV-CAG-co-hu-SFMD deposited on May 16^{th}, 2011, under access number DSM 24817 at the DSMZ - Deutsche Sammlung von Mikroorganismen und Zellkulturen, as described in WO2011154520. Said plasmid contains cDNA of optimized human sulfamidase (SEQ ID NO: 1) under the control of the ubiquitous CAG promoter and flanked by AAV2 ITRs.

As mentioned before, the original AAV genes, Rep and Cap, are provided on a separate plasmid *in-trans.* Without the presence of the ITR regions on this plasmid, the Rep and Cap genes will not be packaged into the viral capsid during production and will remain within the parent cell for the duration of the production. Usually, the required adenovirus helper genes used in rAAV production are provided on another separate plasmid. Once all components are transfected together into a packaging cell line, AAV particles are assembled using the cell's cellular machineries.

AAV vectors are then produced by triple transfection of suitable mammalian cells, for example HEK293 cells, and purified using an optimized purification protocol with double cesium chloride gradient ultracentrifugation that results in vector preps of high purity with neglectful amounts of empty capsids (Ayuso *et al,* 2010).

In a preferred embodiment, the AAV first and terminal repeats of the first vector are ITRs from the AAV serotype 2. In another preferred embodiment, the AAV *rep* genes of the second vector are from the AAV serotype 2. In yet another preferred embodiment, the AAV cap genes of the second vector are from the AAV serotypes 1, 2, 5, 7, 8 or 9. More preferably, the AAV cap genes of the second vector are from the AAV serotype 9. In another preferred embodiment, the competent cells are HEK293 cells.

In another particular embodiment, the vectors of the invention are produced by a manufacturing procedure based on a Bacculovirus Expression System. This system is well known for the skilled person in the art (Urabe *et al,* 2002). This technology allows the production of large quantities of vectors in insect cells Sf9 (*Spodoptera frugiperda*) using serum free culture medium. Briefly, Sf9 cells are coinfected in suspension cultures with three recombinant baculoviruses (a Rep-baculovirus, a VP-baculovirus, and an AAV ITR vector genome baculovirus) and a few days later, rAAV is recovered. The particles produced are indistinguishable from 293 cell-produced rAAV, as determined on the basis of physical properties and biologic activities. Particles produced by either method were composed of similar proteins and nucleic acid.

Pharmaceutical compositions can be supplied as liquid solutions or suspensions, as emulsions, or as solid forms suitable for dissolution or suspension in liquid prior to use. It is also disclosed herein a method for manufacturing the pharmaceutical composition of the invention. This method comprises combining the expression vectors of the invention and a pharmaceutically acceptable vehicle or carrier to facilitate administration to yield the pharmaceutical compositions of the invention.

The carrier is, for instance, water, or a buffered saline solution, with or without a preservative. The pharmaceutical compositions may be lyophilized for resuspension at the time of administration or in solution. In a preferred embodiment, the pharmaceutical composition of the invention is a suspension for injection. The final product is then formulated in a suitable buffer, filled in vials and stored until use.

Neurosurgical administration of the pharmaceutical composition of the invention will involve standard procedures for ICV puncture. Briefly, surgical procedure will be performed in the surgical theatre under general anesthesia. A ventricular puncture assisted by neuronavigation will be performed and the product will be administered.

Safety and tolerability is assessed by physical examination, adverse events, blood and urine laboratory tests, inflammatory response, immune response, ECG, vital signs and imaging tests. The safety focus after vector administration is initially on potential immune response to the product. Peripheral blood mononuclear cells are isolated at entry, at days 15 and 30, at 3 and 6 months and at 1 and 3 years after vector administration for determination of specific effector T cell responses to an array of AAV9 capsid and sulfamidase peptides using a short-term interferon-γ ELISPOT assay, intracellular cytokine staining and lymphocyte proliferation with carboxyfluorescein (CFSE). Humoral immunity to AAV9 is determined by humoral immunity assays.

Having described the invention in general terms, it will be more easily understood by reference to the following examples which are presented as an illustration and are not intended to limit the present invention.

### GENERAL PROCEDURES

### MATERIALS AND METHODS

### AAV vector production

The recombinant adeno-associated viral vector serotype 9 (rAAV9) containing human sulfamidase gene under the control of the CAG promoter (AAV9-CAG-coh-SGSH) is a genetically modified viral vector derived from wild-type AAV. The recombinant vector is composed of the capsid of the serotype 9 of human AAV and a modified genome, containing a codon-optimized version of the human Sulfamidase gene (SGSH) SEQ ID NO: 1, among other elements required for transcription and correct packaging of the active form.

The Drug Substance of the AAV9-CAG-coh-SGSH has been produced under GMP conditions by a manufacturing procedure based on a Bacculovirus Expression System (Urabe *et al,* 2002). This technology allows the production of large quantities of vectors in insect cells Sf9 (*Spodoptera frugiperda*) using serum free culture medium. Briefly, Sf9 cells are coinfected in suspension cultures with three recombinant baculoviruses (a Rep-baculovirus, a VP-baculovirus, and an AAV ITR vector genome baculovirus) and few days later, rAAV is recovered.

The final Drug Product is then formulated in a buffer and filled in vials of 1 mL. The container used for filling is sterile and suitable for the storage of preparations at ≤-70°C.

The final product is a suspension for injection composed of the recombinant viral vector resuspended in sterile formulation buffer (0.001 % Pluronic F68, 180 mM NaCl, 5 mM NaH2PO4.2H20, 5mM Na2HPO4 and H2O).

The identity of the product is determined by genome sequencing (after qPCR amplification) and by viral protein detection by Western Blot to detect the presence of the human sulfamidase transgene and AAV9 viral proteins.

The characterization of the rAAV9 is analysed by visual inspection to determine the appearance of the product, pH and osmolality analysis, and analytical ultracentrifugation to determine the ratio of empty /full viral particles.

The purity of the product is assessed by determining total DNA and total protein quantification by fluorimetry. Moreover, the presence of band corresponding to the AAV capsid proteins is assessed by the separation of the protein content onto a gradient polyacrylamide gel and revealed by silver staining. Additionally, residual host cell and plasmid DNA contamination is determined by qPCR analysis using appropriate genomic DNA or plasmid primers and probes. Residual Triton is determined by HPLSC and residual chromatography ligands AAV9-Poros and residual host cell proteins are determined by ELISA assay.

The titer of the rAAV is determined by vector genome titration by qPCR consisting of the determination of the copy number of viral DNA by targeting a specific sequence. Furthermore, an infectious genome titration by transduction analysed by qPCR demonstrate that the vector is able to infect cells and determines the proportion of viral particles that have been able to go into the cells.

The biological activity (potency) of the product is analysed by a gene expression assay, a test based on the *in vitro* infection of E4 HEK293 cells at MOIs (multiplicity of infection) ranging from 5,000 to 40,000 vg/cels. Total RNA is extracted from cell lysates, retrotranscribed and the amount of vector-derived sulfamidase expression is quantified by qPCR using primers and probes specific for optimized human sulfamidase (that do not detect sulfamidase endogenous to HEK293 cells). The relative sulfamidase expression is determined by calculating the ratio between the expression of sulfamidase and the expression of the internal reference gene used in the qPCR technique.

### AAV administration

The clinical dose administered to the first cohort of patients is 6,8x10¹³ vg/patient.

The pharmaceutical composition is administrated intracerebroventricularly (ICV) to the patients. This method bypasses the blood-brain barrier and other mechanisms that limit drug distribution into the CNS (in particular the brain, spinal cord, etc), allowing high drug concentrations to enter the central compartment. Instillation of drugs directly into the ventricles of the brain.

Surgical procedure is performed in the surgical theatre under general anesthesia. A ventricular puncture assisted by neuronavigation is performed and the product is then administered.

### Safety and tolerability

Safety and tolerability parameters are evaluated at regular time points after product administration and are assessed by comparison to screening / baseline evaluations.

### Inflammatory response assessment in blood.

Immune response: interferon gamma ELISPOT, intracellular cytokine staining and lymphocyte proliferation in peripheral blood mononuclear cells and neutralizing antibodies (NAb) to the AAV9 vector and antibodies to the transgene are quantified in CSF and serum.

Other parameters such as ECG, vital signs and imaging assessments are evaluated after product administration.

### Pharmacodynamic - Efficacy:

All pharmacodynamic and efficacy parameters are evaluated at regular time points after product administration and are assessed by comparison to screening / baseline evaluations.

Sulfamidase enzymatic activity is quantified in cerebrospinal fluid (CSF) and blood (leukocytes and plasma).

### Quantitative analysis of heparan sulfate in plasma, urine and cerebrospinal fluid

Several methods for the quantitative determination of GAGs have been described. Based on the enzymatic digestion with heparitinase, the concentration of heparan sulfate disaccharide in plasma and urine is measured (Tomatsu S *et al.* 2013). Briefly, small amounts of plasma or urine (50 to 200 µL) are analysed after digestion with heparitinase during 3 hours at 37°C. The digested sample is then centrifuged, cleaned up and injected into an UPLC-tandem mass spectrometer (MS/MS) with electrospray ionization (XEVO-TQS, Waters Corp, Milford, MA, USA). Control ranges are achieved using with the same samples for measuring sulfamidase activity.

### Analysis of Heparan-N-Sulfatase (sulfamidase) activity in leukocytes

Leukocytes are isolated from whole blood, previously collected on lithium heparin, using a buffer to lysate the erythrocytes. The leukocyte pellet is resuspended in water and sonicated to break all the cellular membranes and to liberate the lysosomal enzymes. Protein concentration is measured using the BioRad DC Protein Assay (Bio-Rad Laboratories, S.A, Alcobendas, Madrid). Enzymatic activity of heparan-N-sulfatase is determined in triplicate using a fluorimetric assay with 4-Methylumbelliferyl-α-N-sulphoglucosaminide substrate purchased from Enantia (Barcelona, Spain). Fluorescence is measured with a microplate reader (POLARstar Omega, BMG LABTECH, Offenburg, Germany). Results will be expressed as nmol/17 h/mg prot.

The patients are also monitored by physical examination. Further, patients are monitored for possible adverse events.

### Humoral immune response quantification

Humoral immune response to AAV9 and heparan N-sulfatase (transgene) is determined by ELISA for detection of antibodies against AAV9 and against the transgene.

Briefly, for the ELISA assay, blood and CSF samples obtained before and at different time points after ICV administration of the product of the invention are processed as follows: ELISA plates (Poly-Sorp, Nunc, Wiesbaden, Germany) are immobilized overnight at 4°C with the recombinant product (50ul per well) diluted in PBS. Plates are then washed (PBS/0.05% Tween 20), blocked with commercial blocking solution (1h at room temperature) and incubated with 50 µl of test serum diluted in blocking solution (1:50-1:400). After 1 hour incubation at room temperature, plates are washed and incubated for 1 hour at room temperature with a goat anti-human IgG horseradish peroxidase-conjugated antibody diluted in PBS/0.05% Tween 20. After washing, colour is developed with horseradish peroxidase substrate followed by quenching with 1N of H2SO4. Optical density is read at 492nm with a spectrophotometer.

For IgM antibody detection, serum samples are previously treated with an IgG absorbent reagent and a goat anti-human IgM antibody diluted in PBS/0.05% Tween 20 is used as a secondary antibody. Likewise, for IgG subclasses detection, horseradish peroxidase-conjugated antibodies against human IgG1, IgG2, IgG3 or IgG4 diluted in PBS/0.05% Tween 20 is also used.

The test use positive and negative control serums, in which the presence or absence of antibodies against the product is known. The reaction also include a blank well that is incubated only with blocking solution without serum and represents the background value of the assay.

The test is considered valid if the value of the positive control is at least 3 times higher than the value of the negative control and if the blank well is negative. A sample is scored positive if the value is 3 times higher to the value of the negative control. Otherwise the sample is scored negative.

For the sero-neutralization assay serum and CSF samples are serially diluted to determine the titer of neutralizing antibodies against AAV9 in the sample. A starting dilution of 1:1 is used to perform 1/5 dilutions until 1:625. If the sample is neutralizing at a 1:625 dilution, the assay is repeated using higher dilutions to determine the neutralizing titer. If the sample is neutralizing at 1:1 dilution, but not at 1:5 dilution, two-fold dilutions 1:2, 1:4 and 1:8 are tested to determine the neutralizing titer. Each well will be tested in triplicate.

Briefly, 2V6.11 cells are seeded in a 96 well plate at a density of 1.25x10⁴ cells per well and treated with Ponasterone A at a 1:1000 dilution to induce expression of the E4 gene. Each sample dilution is incubated with the luciferase encoding vector during 1h. The wells are inoculated in triplicate with the mixture and after 24h, cells are lysed and luciferase activity is measured with a commercial system (Promega) using a luminometer. The mean of the luciferase activitity of the triplicates of each well is then calculated. The assay is valid if i) the expected values of luciferase activity are obtained in the wells in which the cells are incubated only with the vector (reference value), ii) not significant luciferase activity is obtained in the blank well (cells incubated without vector), iii) the positive control is neutralizing and iv) the negative control is not neutralizing. A sample is scored positive for neutralizing antibodies if the mean of the luciferase activity is ≤50% of the reference value. The titer of neutralizing antibodies for each sample will be the highest dilution showing neutralizing activity.

### RESULTS

A single administration of the pharmaceutical composition into the cerebrospinal fluid (CSF) of MPSIIIA patients, was able to produce steady high levels of SGSH activity in the CSF and other tissues, including liver and serum, in the absence of any adverse events. Also, levels of heparan sulphate have been reduced after treatment.

Safety and tolerability has been assessed by physical examination, adverse events, blood and urine laboratory tests, inflammatory response, immune response, ECG, vital signs and imaging tests. No substantial adverse events have been detected.

Altogether, the results confirm that it is possible to achieve long-term transgene expression, in particular sulfamidase expression, in the CNS using the pharmaceutical composition according to the invention.

While the foregoing invention has been described in some detail for purposes of clarity and understanding, it will be appreciated by one skilled in the art from a reading of this disclosure that various changes in form and detail can be made without departing from the true scope of the invention as defined in the appended claims.

## Claims

1. A pharmaceutical composition comprising a recombinant adeno-associated viral vector comprising a nucleotide sequence as set for in SEQ ID NO: 1 encoding sulfamidase protein of SEQ ID NO: 2 for use as long term treatment, wherein the long term treatment shows an effect that persists for at least three months, of Sanfilippo syndrome type A in a human subject, by means of one single intracerebroventricular administration of the pharmaceutical composition and resulting in prolonged and sustained production of the sulfamidase protein.

2. Pharmaceutical composition for use according to claim 1, wherein said composition is administered to patients with or susceptible to Sanfilippo syndrome type A.

3. Pharmaceutical composition for use according to any one of claims 1 to 2, wherein said composition is administered at a dose of 1x10¹²- 1x10¹⁵ vg/Kg and the administration of the composition:
- reduces at least 10% glycosaminoglycans levels in a sample of a subject at 3 or 6 months after treatment compared to baseline levels, and
- increases at least two-fold enzyme activity levels in a sample of a subject at 3 or 6 months after treatment compared to baseline levels.

4. Pharmaceutical composition for use according to claim 3, wherein said composition is administered at a dose of 1x10¹³- 1x10¹⁵ vg/Kg.

5. Pharmaceutical composition for use according to claim 4, wherein said composition is administered at a dose of 1x10¹³- 3x10¹⁴ vg/Kg.

6. Pharmaceutical composition for use according to any one of claims 3 to 5 wherein said glycosaminoglycans levels are heparan sulfate levels.

7. A method for evaluating and/or monitoring the efficacy of a long term treatment, wherein the long term treatment shows an effect that persists for at least three months, of Sanfilippo syndrome type A in a human subject after one single intracerebroventricular administration of a pharmaceutical composition comprising a recombinant adeno-associated viral vector comprising a nucleotide sequence as set for in SEQ ID NO: 1 encoding sulfamidase protein of SEQ ID NO: 2, comprising measuring sulfamidase enzymatic activity in a sample of the treated subject at least three months after administration.

8. Method according to claim 7, wherein measuring sulfamidase enzymatic activity in a sample of the treated subject is performed at least six months after administration.

9. Method according to claims 7 or 8, wherein said sample is a cerebra-spinal fluid sample or a serum sample.

## Patentansprüche

1. Pharmazeutische Zusammensetzung, umfassend einen rekombinanten adenoassoziierten viralen Vektor, der eine Nukleotidsequenz wie in SEQ ID NO: 1 umfasst, die das Sulfamidase-Protein von SEQ ID NO: 2 codiert, zur Verwendung als Langzeitbehandlung, wobei die Langzeitbehandlung eine Wirkung zeigt, die mindestens drei Monate anhält, des Sanfilippo-Syndroms Typ A bei einem menschlichen Patienten, mittels einer einzigen intrazerebroventrikulären Verabreichung der pharmazeutischen Zusammensetzung und die zu einer verlängerten und anhaltenden Produktion des Sulfamidase-Proteins führt.

2. Pharmazeutische Zusammensetzung zur Verwendung nach Anspruch 1, wobei die Zusammensetzung Patienten verabreicht wird, die an Sanfilippo-Syndrom Typ A leiden oder dafür anfällig sind.

3. Pharmazeutische Zusammensetzung zur Verwendung nach einem der Ansprüche 1 bis 2, wobei die Zusammensetzung in einer Dosis von 1x10¹²-1x10¹⁵ vg/Kg verabreicht wird und die Verabreichung der Zusammensetzung:
- den Glykosaminoglykanspiegel in einer Probe eines Patienten 3 oder 6 Monate nach der Behandlung im Vergleich zum Ausgangswert um mindestens 10 % verringert und
- die Niveaus an Enzymaktivität in einer Probe eines Patienten 3 oder 6 Monate nach der Behandlung im Vergleich zum Ausgangswert um mindestens das Zweifache erhöht.

4. Pharmazeutische Zusammensetzung zur Verwendung nach Anspruch 3, wobei die Zusammensetzung in einer Dosis von 1x10¹³- 1x10¹⁵ vg/Kg verabreicht wird.

5. Pharmazeutische Zusammensetzung zur Verwendung nach Anspruch 4, wobei die Zusammensetzung in einer Dosis von 1x10¹³- 3x10¹⁴ vg/Kg verabreicht wird.

6. Pharmazeutische Zusammensetzung zur Verwendung nach einem der Ansprüche 3 bis 5, wobei die Glykosaminoglykanspiegel Heparansulfatspiegel sind.

7. Verfahren zum Bewerten und/oder Überwachen der Wirksamkeit einer Langzeitbehandlung, wobei die Langzeitbehandlung eine Wirkung zeigt, die mindestens drei Monate anhält, des Sanfilippo-Syndroms Typ A bei einem menschlichen Patienten nach einer einzigen intrazerebroventrikulären Verabreichung einer pharmazeutischen Zusammensetzung, die einen rekombinanten adenoassoziierten viralen Vektor umfasst, der eine Nukleotidsequenz wie in SEQ **ID** NO: 1 umfasst, die das Sulfamidase-Protein der SEQ **ID** NO: 2 codiert, umfassend die Messung der enzymatischen Aktivität der Sulfamidase in einer Probe des behandelten Patienten mindestens drei Monate nach der Verabreichung.

8. Verfahren nach Anspruch 7, wobei die Messung der enzymatischen Aktivität der Sulfamidase in einer Probe des behandelten Patienten mindestens sechs Monate nach der Verabreichung durchgeführt wird.

9. Verfahren nach Anspruch 7 oder 8, wobei die Probe eine Probe von Gehirn-Rückenmarks-Flüssigkeit oder eine Serumprobe ist.

## Revendications

1. Composition pharmaceutique comprenant un vecteur viral adéno-associé recombinant comprenant une séquence nucléotidique telle que définie dans SEQ ID No : 1 codant pour la protéine sulfamidase de SEQ ID No : 2 pour une utilisation en tant que traitement à long terme, dans laquelle le traitement à long terme montre un effet qui persiste pendant au moins trois mois, de la maladie de Sanfilippo de type A chez un sujet humain, au moyen d'une seule administration intracérébroventriculaire de la composition pharmaceutique et entraînant une production prolongée et soutenue de la protéine sulfamidase.

2. Composition pharmaceutique pour une utilisation selon la revendication 1, dans laquelle ladite composition est administrée à des patients atteints de la maladie de Sanfilippo de type A ou susceptibles de l'être.

3. Composition pharmaceutique pour une utilisation selon l'une quelconque des revendications 1 à 2, dans laquelle ladite composition est administrée à une dose de 1x10¹² à 1x10¹⁵ vg/Kg et l'administration de la composition :
- réduit d'au moins 10 % les niveaux de glycosaminoglycanes dans un échantillon d'un sujet 3 ou 6 mois après le traitement par rapport aux niveaux de base, et
- multiplie au moins par deux les niveaux d'activité enzymatique dans un échantillon d'un sujet 3 ou 6 mois après le traitement par rapport aux niveaux de base.

4. Composition pharmaceutique pour une utilisation selon la revendication 3, dans laquelle ladite composition est administrée à une dose de 1x10¹³ à 1x10¹⁵ vg/Kg.

5. Composition pharmaceutique pour une utilisation selon la revendication 4, dans laquelle ladite composition est administrée à une dose de 1x10¹³ à 3x10¹⁴ vg/Kg.

6. Composition pharmaceutique pour une utilisation selon l'une quelconque des revendications 3 à 5 dans laquelle lesdits niveaux de glycosaminoglycanes sont des niveaux d'héparane sulfate.

7. Procédé d'évaluation et/ou de contrôle de l'efficacité d'un traitement à long terme, dans lequel le traitement à long terme montre un effet qui persiste pendant au moins trois mois, de la maladie de Sanfilippo de type A chez un sujet humain après une seule administration intracérébroventriculaire d'une composition pharmaceutique comprenant un vecteur viral adéno-associé recombinant comprenant une séquence nucléotidique telle que définie dans SEQ **ID** No : 1 codant pour la protéine sulfamidase de SEQ **ID** No : 2, comprenant la mesure de l'activité enzymatique de la sulfamidase dans un échantillon du sujet traité au moins trois mois après l'administration.

8. Procédé selon la revendication 7, dans lequel la mesure de l'activité enzymatique de la sulfamidase dans un échantillon du sujet traité est effectuée au moins six mois après l'administration.

9. Procédé selon les revendications 7 ou 8, dans lequel ledit échantillon est un échantillon de liquide cérébrospinal ou un échantillon de sérum.
